# EUROPÄISCHE PATENTSCHRIFT

(11) **EP 0 449 912 B1**
(45) Veröffentlichungstag und Bekanntmachung des Hinweises auf die Patenterteilung: **02.03.1994**
(21) Anmeldenummer: 90900819.5
(22) Anmeldetag: 15.12.1989
(51) Int. Cl.: C07C 69/54, C07C 67/08

(54) **VERFAHREN ZUR VERBESSERTEN HERSTELLUNG VON (METH)ACRYLSÄUREESTERN MEHRWERTIGER ALKOHOLE (II)**
IMPROVED PROCESS FOR PRODUCING ESTERS OF (METH)ACRYLIC ACID OF POLYHYDRIC ALCOHOLS (II)
PROCEDE DE PRODUCTION D'ESTERS D'ACIDE (METH)ACRYLIQUE D'ALCOOLS POLYVALENTS (II)

(30) Priorität: 24.12.1988 DE 3843938
(43) Veröffentlichungstag der Anmeldung: 09.10.1991
(73) Patentinhaber: Henkel Kommanditgesellschaft auf Aktien, 40191 Düsseldorf (DE)
(72) Erfinder: RITTER, Wolfgang, D-5657 Haan (DE); SITZ, Hans-Dieter, D-4049 Rommerskirchen (DE); SPEITKAMP, Ludwig, D-4000 Düsseldorf 13 (DE)
(74) Vertreter: von Kreisler, Alek, Dipl.-Chem.
(86) Internationale Anmeldenummer: EP8901550
(87) Internationale Veröffentlichungsnummer: WO9007487

(56) Entgegenhaltungen:
- US-A- 3 887 609

## Beschreibung

Die Erfindung betrifft ein Verfahren zur Herstellung von polyfunktionellen Estern der Acrylsäure und/oder der Methacrylsäure mit mehrwertigen Alkoholen - im folgenden auch als (Meth)acrylsäureester bzw. Poly(Meth)acrylsäureester bezeichnet - durch Umsetzung der Reaktanten in Gegenwart saurer Veresterungskatalysatoren unter Zusatz von Polymerisationsinhibitoren zum Reaktionsgemisch.

(Meth)acrylsäureester mehrwertiger Alkohole insbesondere aus der Gruppe der 2- bis 4wertigen aliphatischen gesättigten Alkohole und deren Oxalkylierungsprodukte finden zunehmende Bedeutung als hochreaktive Bestandteile in strahlenhärtenden Systemen. Solche polyfunktionellen (Meth)acrylsäureester können beispielsweise als Lackrohstoffe für die Elektronenstrahlhärtung oder als Bestandteil von UV-Licht-härtenden Druckfarben oder entsprechenden Überzugslacken, Spachtel, Form- oder Vergußmassen sowie in Klebstoffen, insbesondere anaerob härtenden Klebstoffen Verwendung finden. Ihre Herstellung ist allerdings nicht problemlos. Gefordert werden insbesondere farblose Produkte mit geringer Säurezahl und hoher Lagerstabilität, die praktisch auch keinen Eigengeruch aufweisen. Eine destillative Reinigung der (Meth)acrylsäureester der hier betroffenen Art scheidet in aller Regel aufgrund ihres hohen Molekulargewichtes und ihrer hohen Reaktivität aus. Die Produkte sollen also unmittelbar als möglichst farblose Reaktionsprodukte der Veresterung anfallen. Die Durchführung der Veresterungsreaktion fordert die Mitverwendung hochwirksamer Inhibitoren, die ihrerseits keine unerwünschten Nebenreaktionen, beispielsweise Verfärbungen, auslösen.

Zur Herstellung solcher polyfunktioneller (Meth)acrylsäureester mehrwertiger Alkohole besteht umfangreiche Vorliteratur. Verwiesen sei insbesondere auf die deutsche Offenlegungsschrift 29 13 218 und die darin zitierte einschlägige Literatur. So ist es aus der DE-AS 12 67 547 und aus der Zeitschrift "Chem. and Ind." 18 (1970), 597, bekannt, polyfunktionelle (Meth)acrylsäureester durch azeotrope Veresterung der (Meth)acrylsäure mit mehrwertigen Alkoholen in Gegenwart von azeotropen Schleppmitteln sowie von sauren Katalysatoren und Polymerisationsinhibitoren herzustellen. Als Polymerisationsinhibitoren wurden vorgeschlagen Phenole, Phenolderivate, Kupfer, Kupferverbindungen oder Phenothiazin. Als saure Katalysatoren werden organische oder anorganische Säuren oder saure Ionenaustauscher eingesetzt, wobei p-Toluolsulfonsäure und Schwefelsäure bevorzugt sein können. Die Veresterung erfolgt insbesondere bei Temperaturen von 40 bis 120 °C. Geeignete azeotrope Schleppmittel für die Entfernung des Reaktionswassers sind aliphatische oder cycloaliphatische oder aromatische Kohlenwasserstoffe bzw. deren Gemische mit Siedebereichen innerhalb der angegebenen Temperaturgrenzen.

In der genannten DE-OS 29 13 218 wird vorgeschlagen, die azeotrope Veresterung in Gegenwart mindestens eines organischen Esters der phosphorigen Säure zusätzlich zu einem mitverwendeten Inhibitor auf Phenolbasis durchzuführen. Zwingend wird allerdings auch hier die Mitverwendung mindestens eines aliphatischen, cycloaliphatischen und/oder aromatischen Kohlenwasserstoffs mit einem Siedepunkt im Bereich von 40 bis 120 °C gefordert. Das entstehende Reaktionswasser soll mittels dieser Schleppmittel azeotrop ausgekreist werden. Die Reaktionsdauer wird nach den Beispielen dieser Druckschrift mit 10 bis 18 Stunden angesetzt.

Die Erfindung geht von der Aufgabe aus, Reaktionsbedingungen für die hier betroffene Veresterungsreaktion zu ermitteln, die einerseits zu einer substantiellen Abkürzung der Reaktionsdauer führen können, andererseits aber die Qualität der entstehenden Veresterungsprodukte und insbesondere die hohe Farbqualität nicht negativ beeinflussen. Die Erfindung will weiterhin darauf verzichten können, vergleichsweise komplexe Inhibitorsysteme derart einsetzen zu müssen, wie sie in der genannten DE-OS 29 13 218 beschrieben sind. Es soll dabei erfindungsgemäß auch möglich sein, den im praktischen Einsatz gewünschten Applikationsinhibitor der hier betroffenen hochreaktiven Systeme gleichzeitig schon als Reaktionsinhibitor bei der Synthese der polyfunktionellen (Meth)acrylsäureester einzusetzen.

Die erfindungsgemäße Lehre beschäftigt sich insbesondere mit den Problemen, die bei Umsetzungen der geschilderten Art dann auftreten, wenn nicht substituierte Phenolverbindungen und hier insbesondere das Hydrochinon als alleinige oder wenigstens als wesentliche Inhibitorkomponenten zum Einsatz kommen. Die Verwendung von Hydrochinon als in der Praxis eingeführter Polymerisationsinhibitor kann aus dem Applikationsgesichtspunkt wünschenswert sein. Als Reaktionsinhibitor verwendet zeigt sich, daß insbesondere Schwierigkeiten bezüglich der Farbe des anfallenden Reaktionsproduktes bestehen. Die einfache Behandlung solcher stark farbiger Reaktionsprodukte mit beispielsweise Aktivkohle als Entfärbungsmittel führt nicht zu den erwünschten Aufhellungen. Gleichwohl hat sich überraschenderweise gezeigt, daß durch das Arbeiten im Sinne der nachfolgend geschilderten erfindungsgemäßen Lehre hier wirkungsvoll Abhilfe geschaffen werden kann.

Die technische Lösung der Erfindung geht in einer bevorzugten Ausführungsform weiterhin von der Erkenntnis aus, daß vergleichsweise hochreine Veresterungsprodukte als unmittelbare Verfahrensprodukte selbst dann erhalten werden können, wenn auf die Mitverwendung von Verdünnungsmitteln bzw. azeotropen Schleppmitteln verzichtet wird und daß es unter den Bedingungen des lösungsmittelfreien Arbeitens sogar möglich ist, vergleichsweise schärfere Veresterungsbedingungen einzusetzen, die zu einer beträchtlichen Abkürzung der Reaktionszeit führen. Voraussetzung hierfür ist insbesondere, daß unter den nachfolgenden geschilderten Verfahrensbedingungen gearbeitet wird.

Gegenstand der Erfindung ist dementsprechend ein Verfahren zur Herstellung von (Meth)acrylsäureestern mehrwertiger Alkohole durch deren Umsetzung mit Acrylsäure und/oder Methacrylsäure in Gegenwart von sauren Veresterungskatalysatoren unter Zusatz von Polymerisationsinhibitoren zum Reaktionsgemisch. Das neue Verfahren ist dadurch gekennzeichnet, daß man beim Einsatz von alpha-ständig nicht substituierten Phenolverbindungen als Inhibitoren die Veresterungsreaktion unter Zusatz von Aktivkohle zum Reaktantengemisch durchführt. Der bevorzugte alpha-ständige nicht substituierte Inhibitor auf Phenolbasis ist das Hydrochinon.

Es hat sich überraschenderweise gezeigt, daß die Mitverwendung von Aktivkohle schon bei der Kondensationsreaktion - und nicht erst in einem nachträglichen Reinigungsschritt - wirkungsvoll die Entstehung unerwünscht stark verfärbter Reaktionsprodukte verhindert. Die Menge der Aktivkohle kann dabei in breiten Grenzen gewählt werden. Sie soll bevorzugt ein mehrfaches der Menge der als Inhibitor eingesetzten phenolischen Verbindung, also insbesondere des Hydrochinons ausmachen. Wenn auch schon mit dem 2- bis 10fachen an Aktivkohle befriedigende Farbaufhellungen im Reaktionsprodukt eingestellt werden können, so wird im allgemeinen doch die Aktivkohle in noch größeren Mengen beispielsweise im Bereich des 10- bis 100fachen und zweckmäßigerweise im Bereich von etwa dem 20- bis 60fachen der Hydrochinonmenge eingesetzt. Es war nicht zu erwarten, daß bei Verwendung solcher vergleichsweise großer Mengen an Aktivkohle keine substantielle Beeinträchtigung der Inhibitorwirkung gegenüber unerwünschten vorzeitigen Polymerisationserscheinungen eintritt. Offenbar wirkt sich die bekannte Adsorptionsbereitschaft der großen Oberfläche der Aktivkohle vorwiegend gegenüber im Reaktionsgeschehen entstehenden farblichen Verunreinigungen aus und entfernt diese weitgehend selektiv aus dem Reaktionsgemisch, ohne dabei gleichzeitig die Wirkung der als Inhibitor an sich in nur geringer Menge eingesetzten phenolischen Verbindungen zu beeinträchtigen.

In einer weiteren wichtigen und bevorzugten Ausführungsform der Erfindung arbeitet man dabei mit bei Reaktionstemperatur flüssigen Reaktionsgemischen, die wenigstens weitgehend frei sind von Lösungs- und/oder azeotropen Schleppmitteln. Auf solche, den Reaktionsablauf und damit eine unerwünschte Verfärbung an sich mildernden Hilfsmitteln kann in dieser Ausführungsform der Erfindung vollständig verzichtet werden. Zweckmäßigerweise wird bei einem solchen Arbeiten das entstehende Kondensationswasser aus der Gasphase des Reaktionsraumes abgezogen.

Es ist dabei erfindungsgemäß weiterhin bevorzugt, den Reaktionsraum mit einem Gasstrom zu durchspülen und diesen Gasstrom insbesondere dazu zu benutzen, das Kondensationswasser aus der Veresterungsreaktion aus dem Reaktor auszuschleusen. Bevorzugt wird dabei mit einem Gasstrom gearbeitet, der einen beschränkten Anteil an freiem Sauerstoff enthält. In Abhängigkeit von den jeweils gewählten Verfahrensbedingungen kann Luft oder ein an Sauerstoff verarmtes Gasgemisch eingesetzt werden. Ein Beispiel der zuletzt genannten Art sind Stickstoff/Luft-Gemische. In aller Regel wird allerdings ein gewisser Gehalt an freiem Sauerstoff in dieser der Reaktionsmischung zugeführten Gasphase gewünscht. Diese beschränkten Sauerstoffmengen aktivieren in an sich bekannter Weise den Inhibitor während des Reaktionsgeschehens.

Der Sauerstoffgehalt des Gasgemisches liegt im allgemeinen bei wenigstens etwa 1 Volumen-% und bevorzugt im Bereich von etwa 2 bis 20 Volumen-%. Aus Gründen der Reaktionssicherheit kann es dabei bevorzugt sein, mit Gehalten an freiem Sauerstoff in der unteren Hälfte des genannten Bereiches, also bis etwa 10 Volumen-% und bevorzugt bis etwa 7 Volumen-% zu arbeiten. Der Gasstrom wird in einer bevorzugten Ausführungsform der Erfindung in das flüssige Reaktionsgemisch eingespeist und kann dieses beispielsweise feinverteilt durchperlen. Es wird dabei zweckmäßigerweise mit begrenzten Mengen dieses Gasstromes gearbeitet, so daß kein unerwünscht hoher Austrag an Reaktionskomponenten - insbesondere den vergleichsweise leichter flüchtigen Säuren - stattfindet.

Nach Abschluß der Veresterungsreaktion wird die Aktivkohle von dem Reaktionsgemisch abgetrennt. Das kann insbesondere durch Filtration erfolgen. Auf diese Weise gelingt die Herstellung von strahlenhärtbaren polyfunktionellen (Meth)acrylsäureestern mit hoher Reinheit und insbesondere geringer Eigenfarbe auch unter den vergleichsweise scharfen erfindungsgemäß gewählten Verfahrensbedingungen. Die dabei problemlos anfallenden polyfunktionellen (Meth)acrylsäureester zeichnen sich zudem durch hohe Lagerstabilität aus.

Der Polymerisationsinhibitor bzw. gegebenenfalls das Inhibitorgemisch wird dem Reaktionsgemisch üblicherweise in Mengen von 200 bis 10000 ppm und bevorzugt im Bereich von etwa 300 bis 2000 ppm zugesetzt. Die Zahlenangaben beziehen sich dabei jeweils auf das Gewicht des aus (Meth)acrylsäure und polyfunktionellen Alkoholen bestehenden Reaktionsgemisches.

Als zu veresternde Polyalkohole seien beispielsweise genannt: Ethylenglycol, Propylenglycol, Butandiol-1,4, Hexandiol-1,6, Neopentylglycol, Diethylenglycol, Triethylenglycol, Dimethylolpropan, Glycerin, Trimethylolpropan, Trimethylolhexan, Trimethylolethan, Hexantriol-1,3,5 und Pentraerythrit. Erfindungsgemäß kommen als polyfunktionelle Alkohole insbesondere aber auch die Oxalkylierungsprodukte dieser zuvor genannten polyfunktionellen Alkohole in Betracht, wobei hier den Oxethylierungsprodukten und/oder den Oxpropylierungsprodukten besondere Bedeutung zukommt. Kettenverlängerte polyfunktionelle Alkohole dieser Art können beträchtliche Mengen an Polyalkoxidresten enthalten, beispielsweise 1 bis 50 Mol, vorzugsweise etwa 1 bis 20 Mol Ethylenoxid pro g-Äquivalent an Hydroxylgruppen.

Veresterungskatalysatoren für das erfindungsgemäße Herstellungsverfahren sind handelsübliche organische oder anorganische Säuren oder auch saure Ionenaustauscher, wobei den in der Praxis häufig eingesetzten entsprechenden Verbindungen p-Toluolsulfonsäure und Schwefelsäure besondere Bedeutung zukommt. Die Mengen des Veresterungskatalysators liegen beispielsweise im Bereich von 0,1 bis 5 Gew.-% bezogen auf das Veresterungsgemisch.

Die Umsetzung der Reaktanten wird vorzugsweise bei Sumpftemperaturen von wenigstens etwa 90 °C und insbesondere von wenigstens etwa 100 °C durchgeführt. Besonders geeignet ist der Temperaturbereich bis etwa 150 °C. Dabei kann unter Normaldruck, zweckmäßigerweise aber auch unter abgesenktem Druck gearbeitet werden. Beim Arbeiten mit vermindertem Druck kann in einer besonderen Ausführungsform der Druck in Richtung auf niedrigere Drucke stufenweise oder auch kontinuierlich verringert werden.

Durch die Möglichkeit unter vergleichsweise scharfen Veresterungsbedingungen und gleichzeitig vermindertem Druck zu arbeiten, wird die Reaktionsdauer gegenüber bisher beschriebenen Verfahren stark abgekürzt. So können im erfindungsgemäßen Verfahren Umsatzausbeuten von wenigstens 90 % der Theorie und vorzugsweise von wenigstens etwa 94 % der Theorie im Temperaturbereich von etwa 100 bis 140 °C bei einer Reaktionsdauer von nicht mehr als etwa 10 Stunden und vorzugsweise von nicht mehr als etwa 8 Stunden erzielt werden. Gleichwohl fallen die Reaktionsprodukte als hellfarbige oder durch eine einfache Nachbehandlung wirkungsvoll zu reinigende stabilisierte Masse an. Das den sauren Veresterungskatalysator enthaltende Reaktionsrohprodukt wird einer nachfolgenden Neutralisation unterworfen. Diese Neutralisation kann unter bekannten Naßbedingungen, beispielsweise durch Einsatz von wäßrigen Soda- und gegebenenfalls Natriumchlorid enthaltenden Lösungen erfolgen. In einer bevorzugten Ausführungsform wird allerdings das den sauren Katalysator enthaltende Reaktionsrohprodukt einer trockenen Neutralisation unterworfen. Geeignete trockene Neutralisationsmittel sind die Oxide und/oder Hydroxide der Alkalimetalle, der Erdalkalimetalle und/oder des Aluminiums. Besonders geeignet sind zur Trockenneutralisation entsprechende Verbindungen des Magnesiums bzw. des Calciums.

(Meth)acrylsäure und die Alkohole können für die Veresterung in äquivalenten Mengenverhältnissen eingesetzt werden. Bei den mehr als zweiwertigen Alkoholen ist es allerdings auch ohne weiteres möglich, nur einen Teil der Hydroxylgruppen zu verestern. Zur Vollveresterung kann es zweckmäßig sein, die Säurekomponente in leichtem Überschuß über die zur Veresterung der Hydroxylgruppen erforderliche stöchiometrische Menge einzusetzen. Ein solcher Überschuß kann wenigstens etwa 10 Mol-% ausmachen. Nach Abschluß der Reaktion kann gewünschtenfalls zusätzlich ein Inhibitor dem Reaktionsprodukt beigemischt werden.

Treten bei der Herstellung der Reaktionsprodukte unter den erfindungsgemäßen drastischen Veresterungsbedingungen dann doch einmal leichte Farbverschlechterungen im Reaktionsprodukt auf, so lassen sich diese problemlos durch eine Nachbehandlung mit Entfärbungsmitteln beseitigen. Ein geeignetes Entfärbungsmittel ist beispielsweise Aluminiumoxid.

### Beispiele

### Beispiel 1

928,8 g Acrylsäure, 1560,4 g eines propoxylierten Neopentylglycols (OH-Zahl: 460 mg KOH/g Substanz) sowie 87,1 g p-Toluolsulfonsäure und 124,5 g (5 % bezogen auf Acrylsäure + Polyol) A-Kohle wurden in einen 3-Liter-Reaktor eingewogen und mit 2,5 g Hydrochinon (1100 ppm bezogen auf Produktmenge) inhibiert. Unter Durchleiten eines Luft/Stickstoff-Gemisches (5 Vol-% O₂; 20 l/h) wurde die Veresterung unter Wasserabtrennung durchgeführt. Bei einer konstanten Badtemperatur von 143 °C und einer maximalen Sumpftemperatur von 135 °C betrug die Veresterungszeit 5 Stunden. Der Ansatz wurde abgekühlt und die A-Kohle mit einer Druckfilternutsche abfiltriert.

| Rohprodukt: | |
|---|---|
| Säurezahl: | 34 mg KOH/g |
| OH-Zahl: | 10 mg KOH/g |
| Umsatz: | 96,7 % |
| Gardner Farbzahl: | < 1 |
| Viskosität: | 92 mPas |

Das Rohprodukt wurde mit 4 Liter wäßriger 16 Gew.-% NaCl/4 Gew.-% NaHCO₃-Lösung gewaschen, mit 200 ppm Hydrochinonmonomethylether nachinhibiert und im Vakuum bei 40 mbar und 80 °C 3 Stunden getrocknet und anschließend mit einer Druckfilternutsche filtriert.

| Produkt: | |
|---|---|
| Säurezahl: | < 1 mg KOH/g |
| OH-Zahl: | < 15 mg KOH/g |
| Gardner Farbzahl: | 3 |

### Vergleichsbeispiel 1

Beispiel 1 wurde wiederholt mit der Änderung, daß auf die Zugabe der A-Kohle verzichtet wurde.

| Rohprodukt: | |
|---|---|
| Säurezahl: | 39 mg KOH/g |
| OH-Zahl: | < 15 mg KOH/g |
| Umsatz: | 97,1 % |
| Gardner Farbzahl: | 12 |
| Viskosität: | 90 mPas |

Das Rohprodukt wurde analog zu Beispiel 1 aufgearbeitet.

| Produkt: | |
|---|---|
| Säurezahl: | < 1 mg KOH/g |
| OH-Zahl: | < 15 mg KOH/g |
| Gardner Farbzahl: | 8 - 9 |

### Beispiel 2

Die Veresterung wurde wie in Beispiel 1 durchgeführt mit der Änderung, daß die A-Kohle von 124,5 g auf 24,9 g (1 % bezogen auf Acrylsäure + Polyol) reduziert wurde.

| Rohprodukt: | |
|---|---|
| Säurezahl: | 30 mg KOH/g |
| OH-Zahl: | < 15 mg KOH/g |
| Umsatz: | 97,3 % |
| Gardner Farbzahl: | 6 - 7 |

Das Rohprodukt wurde analog zu Beispiel 1 aufgearbeitet.

| Produkt: | |
|---|---|
| Säurezahl: | < 1 mg KOH/g |
| OH-Zahl: | < 15 mg KOH/g |
| Gardner Farbzahl: | 5 - 6 |

**Tabelle 1**

| Abhängigkeit der Gardner Farbzahl von der A-Kohlemenge bei unterschiedlichen Hydrochinonkonzentrationen für die Veresterung von Acrylsäure mit propoxyliertem Neopentylglycol. | | |
|---|---|---|
| A-Kohlemenge xfacher Überschuß bez. auf Hydrochinon | Gardner Farbzahl des Rohproduktes mit 500 ppm Hydrochinon | Gardner Farbzahl des Rohproduktes mit 1000 ppm Hydrochinon |
| 100 | < 1 | - |
| 80 | < 1 | - |
| 60 | < 1 | - |
| 50 | - | < 1 |
| 40 | 1 - 2 | < 1 |
| 30 | - | 1 - 2 |
| 20 | 5 | 5 |
| 10 | - | 6 - 7 |
| 0 | 12 | 12 |

### Beispiel 3

In einem 3-Liter-Reaktor wurden 1198,8 g Acrylsäure, 1362,3 g eines ethoxylierten Trimethylolpropan (OH-Zahl: 680 mg KOH/g Substanz), 89,6 g p-Toluolsulfonsäure, 128,1 g (5 Gew.-% bezogen auf Acrylsäure + Polyol) A-Kohle sowie 2,56 g Hydrochinon (1100 ppm bezogen auf Produktmenge) eingewogen.

Die Veresterung wurde unter Durchleiten eines Luft/Stickstoff-Gemisches (5 Vol-% O₂; 20 l/h) und unter Wasserabtrennung durchgeführt. Bei einer konstanten Badtemperatur von 143 °C und einer maximalen Sumpftemperatur von 135 °C betrug die Veresterungszeit 5 Stunden. Der Ansatz wurde abgekühlt und die A-Kohle mit Hilfe einer Druckfilternutsche abfiltriert.

| Rohprodukt: | |
|---|---|
| Säurezahl: | 25,3 mg KOH/g |
| OH-Zahl: | 12 mg KOH/g |
| Umsatz: | 96,3 % |
| Gardner Farbzahl: | 3 |
| Viskosität: | 78,9 mPas |

Das Rohprodukt wurde mit 4 Liter wäßriger 16 Gew.-% NaCl/4 Gew.-% NaHCO₃-Lösung gewaschen, mit 200 ppm Hydrochinonmonomethylether nachinhibiert und im Vakuum bei 40 mbar und 80 °C 3 Stunden getrocknet und mit Hilfe einer Druckfilternutsche filtriert.

| Produkt: | |
|---|---|
| Säurezahl: | < 1 mg KOH/g |
| OH-Zahl: | < 15 mg KOH/g |
| Gardner Farbzahl: | 4 |

### Vergleichsbeispiel 2

Beispiel 3 wurde wiederholt mit der Änderung, daß auf die Zugabe der A-Kohle verzichtet wurde.

| Rohprodukt: | |
|---|---|
| Säurezahl: | 27 mg KOH/g |
| OH-Zahl: | < 15 mg KOH/g |
| Umsatz: | 96,4 % |
| Gardner Farbzahl: | 11 - 12 |

Das Rohprodukt wurde analog zu Beispiel 4 aufgearbeitet.

| Produkt: | |
|---|---|
| Säurezahl: | < 1 mg KOH/g |
| OH-Zahl: | < 15 mg KOH/g |
| Gardner Farbzahl: | 7 |

### Beispiel 4

Die Veresterung wurde wie in Beispiel 3 durchgeführt mit der Änderung, daß die A-Kohlemenge von 128,1 g auf 25,6 g (1 Gew.-% bezogen auf Acrylsäure + Polyol) reduziert wurde.

| Rohprodukt: | |
|---|---|
| Säurezahl: | 28,3 mg KOH/g |
| OH-Zahl: | < 15 mg KOH/g |
| Umsatz: | 95,4 % |
| Gardner Farbzahl: | 8 |

Das Rohprodukt wurde analog zu Beispiel 4 aufgearbeitet.

| Produkt: | |
|---|---|
| Säurezahl: | < 1 mg KOH/g |
| OH-Zahl: | < 15 mg KOH/g |
| Gardner Farbzahl: | 6 - 7 |

**Tabelle 2**

| Abhängigkeit der Gardner Farbzahl von der A-Kohlemenge bei unterschiedlichen Hydrochinonkonzentrationen für die Veresterung von Acrylsäure mit ethoxyliertem Trimethylolpropan. | | |
|---|---|---|
| A-Kohlemenge xfacher Überschuß bez. auf Hydrochinon | Gardner Farbzahl des Rohproduktes mit 500 ppm Hydrochinon | Gardner Farbzahl des Rohproduktes mit 1000 ppm Hydrochinon |
| 100 | 2 | - |
| 80 | 2 | - |
| 60 | 3 | - |
| 50 | - | 3 |
| 40 | 5 | 3 |
| 30 | - | 3 - 4 |
| 20 | 8 | 6 - 7 |
| 10 | - | 8 |
| 0 | 11 - 12 | 11 - 12 |

## Patentansprüche

1. Verfahren zur Herstellung von (Meth)acrylsäureestern mehrwertiger Alkohole durch deren Umsetzung mit Acrylsäure und/oder Methacrylsäure in Gegenwart von sauren Veresterungskatalysatoren unter Zusatz von Polymerisationsinhibitoren zum Reaktionsgemisch, dadurch gekennzeichnet, daß man beim Einsatz von nicht substituierten Phenolverbindungen als Inhibitoren die Veresterungsreaktion unter Zusatz von Aktivkohle zum Reaktantengemisch durchführt.

2. Verfahren nach Anspruch 1, dadurch gekennzeichnet, daß man Hydrochinon als Inhibitor verwendet und dabei die Aktivkohle bevorzugt im Bereich des mehrfachen, insbesondere im Bereich des 10- bis 100fachen, zweckmäßig im Bereich des 20- bis 60fachen der Hydrochinonmenge einsetzt.

3. Verfahren nah Ansprüchen 1 und 2, dadurch gekennzeichnet, daß man mit bei Reaktionstemperatur flüssigen Reaktionsgemischen arbeitet, die wenigstens weitgehend frei von Lösungs- und/oder azeotropen Schieppmitteln sind, wobei bevorzugt das entstehende Kondensationswasser aus der Gasphase des Reaktionsraumes abgezogen wird.

4. Verfahren nach Ansprüchen 1 bis 3, dadurch gekennzeichnet, daß man den Reaktionsraum mit einem freien Sauerstoff enthaltenden Gasstrom durchspült.

5. Verfahren nach Ansprüchen 1 bis 4, dadurch gekennzeichnet, daß man Luft oder O₂-abgemagerte Gasgemische, insbesondere Stickstoff/Luft-Gemische zum Durchspülen des Reaktionsraumes und Austrag des Reaktionswassers einsetzt.

6. Verfahren nach Ansprüchen 1 bis 5, dadurch gekennzeichnet, daß die Veresterung bei Sumpftemperaturen von wenigstens etwa 90 °C, vorzugsweise von wenigstens etwa 100 °C und insbesondere im Bereich bis etwa 150 °C durchgeführt wird, wobei bevorzugt wenigstens absatzweise bei vermindertem, gegebenenfalls stufenweise zunehmend vermindertem Druck gearbeitet wird.

7. Verfahren nach Ansprüchen 1 bis 6, dadurch gekennzeichnet, daß die Inhibitoren in Mengen von 200 bis 10000 ppm, bevorzugt im Bereich von 300 bis 2000 ppm - jeweils bezogen auf das Gewicht des Reaktionsgemisches - eingesetzt werden.

8. Verfahren nach Ansprüchen 1 bis 7, dadurch gekennzeichnet, daß die Reaktion auf einen Umsatz von wenigstens 90 % der Theorie, vorzugsweise von wenigstens 94 % der Theorie geführt wird, wobei bevorzugt im Temperaturbereich von etwa 100 bis 140 °C, gegebenenfalls unter Vakuum für eine Reaktionsdauer von nicht mehr als 10 Stunden, insbesondere von nicht mehr als 8 Stunden gearbeitet wird.

9. Verfahren nach Ansprüchen 1 bis 8, dadurch gekennzeichnet, daß das Reaktionsrohprodukt einer trockenen Neutralisation - bevorzugt mit Oxiden und/oder Hydroxiden der Alkalimetalle, der Erdalkalimetalle und/oder des Aluminiums - unterworfen wird.

10. Verfahren nach Ansprüchen 1 bis 9, dadurch gekennzeichnet, daß die primär anfallenden Reaktionsprodukte einer abschließenden Behandlung mit Entfärbungsmitteln unterworfen werden.

## Claims

1. A process for the production of (meth)acrylic acid esters of polyhydric alcohols by the reaction thereof with acrylic acid and/or methacrylic acid in the presence of acidic esterification catalysts with the addition of polymerization inhibitors to the reaction mixture, characterized in that, where unsubstituted phenolic compounds are used as inhibitors, the esterification reaction is carried out with addition of activated carbon to the reaction mixture.

2. The process according to claim 1, characterized in that hydroquinone is used as the inhibitor and the activated carbon is preferably used in a quantity which is several times larger and preferably is within the range of from 10 to 100 times, and expediently within the range of from 20 to 60 times, the quantity of the hydroquinone.

3. The process according to claims 1 and 2, characterized in that the reaction mixtures used are liquid at room temperature and are at least substantially free from solvents and/or azeotropic entraining agents, wherein the resulting water of condensation is removed from the gaseous phase of the reaction volume.

4. The process according to claims 1 to 3, characterized in that the reaction volume is purged with a gas stream containing free oxygen.

5. The process according to claims 1 to 4, characterized in that air or O₂-depleted gas mixtures, more particularly nitrogen/air mixtures, are used for purging the reaction volume and for removing the water of reaction.

6. The process according to claims 1 to 5, characterized in that the esterification reaction is carried out at bottoms temperatures of at least about 90 °C, and preferably of at least about 100 °C and especially in the range of up to about 150 °C, wherein the reaction is preferably carried out at least batchwise under reduced pressure, and optionally under a pressure increasingly reduced in steps.

7. The process according to claims 1 to 6, characterized in that the inhibitors are used in quantities of from 200 to 10,000 ppm, and preferably in quantities of from 300 to 2,000 ppm, each based on the weight of the reaction mixture.

8. The process according to claims 1 to 7, characterized in that the reaction is conducted to reach a yield of at least 90% of the theoretical yield, and preferably of at least 94% of the theoretical yield, while it is preferred to operate in a temperature range of from about 100 to 140 °C, optionally *in vacuo*, for a period of reaction of not more than 10 hours and, in particular, of not more than 8 hours.

9. The process according to claims 1 to 8, characterized in that the crude reaction product is subjected to a dry neutralization, preferably with oxides and/or hydroxides of the alkali metals, the alkaline earth metals and/or of aluminium.

10. The process according to claims 1 to 9, characterized in that the reaction products primarily obtained are subjected to a final treatment with decolorizing agents.

## Revendications

1. Procédé d'obtention d'esters d'acide (méth)acrylique et d'alcools plurivalents par leur réaction avec l'acide acrylique et/ou l'acide méthacrylique en présence de catalyseurs d'estérification acides tout en ajoutant des inhibiteurs de polymérisation au mélange réactionnel, caractérisé en ce que l'on effectue la réaction d'estérification tout en ajoutant du charbon actif au mélange de réactifs lors de l'utilisation de composés phénoliques non substitués en tant qu'inhibiteurs.

2. Procédé selon la revendication 1, caractérisé en ce que l'on utilise l'hydroquinone comme inhibiteur et pour cela on met en jeu le charbon actif de préférence au niveau d'un multiple, en particulier au niveau de 10 à 100 fois, d'une manière appropriée au niveau de 20 à 60 fois, de la quantité d'hydroquinone.

3. Procédé selon les revendications 1 et 2, caractérisé en ce que l'on travaille avec des mélanges réactionnels liquides à la température de réaction, qui sont au moins largement dépourvus de solvants et/ou d'agents d'entraînement azéotropiques, dans lequel de préférence l'eau de condensation qui s'est formée est chassée de la phase gazeuse de l'espace de réaction.

4. Procédé selon les revendications 1 à 3, caractérisé en ce que l'on rince complètement l'espace de réaction avec un courant gazeux contenant de l'oxygène libre.

5. Procédé selon les revendications 1 à 4, caractérisé en ce que l'on met en oeuvre de l'air ou des mélanges de gaz appauvris en O₂, en particulier des mélanges Azote/air pour le rinçage complet de l'espace de réaction et l'évacuation de l'eau de réaction.

6. Procédé selon les revendications 1 à 5, caractérisé en ce que l'estérification est effectuée à une température de pot d'au moins environ 90°C, de préférence d'au moins environ 100°C et en particulier dans la zone allant jusqu'à environ 150°C, dans lequel de préférence on travaille au moins de manière intermittente sous pression réduite, le cas échéant sous pression réduite progressivement par paliers.

7. Procédé selon les revendications 1 à 6, caractérisé en ce que l'on met en jeu les inhibiteurs en quantités de 200 à 10 000 ppm de préférence dans la zone de 300 à 2 000 ppm, respectivement rapporté au poids du mélange réactionnel.

8. Procédé selon les revendications 1 à 7, caractérisé en ce que la réaction est conduite jusqu'à une conversion d'au moins 90 % de la théorie, de préférence d'au moins 94 % de la théorie, dans lequel de préférence on travaille dans une plage de températures d'environ 100 à 140°C, le cas échéant sous vide, pour une durée de réaction qui n'est pas supérieure à 10 heures, en particulier pas plus de 8 heures.

9. Procédé selon les revendications 1 à 8, caractérisé en ce que le produit brut de la réaction est soumis à une neutralisation à sec, de préférence avec des oxydes et/ou des hydroxydes de métaux alcalins, de métaux alcalino-terreux et/ou d'aluminium.

10. Procédé selon les revendications 1 à 9, caractérisé en ce que les produits de réaction primaire qui se forment sont soumis à un traitement final avec des agents de décoloration.
